(19) Europäisches Patentamt

European Patent Office

Office européen des brevets

(11) **EP 1 397 203 B1**

(12) **EUROPÄISCHE PATENTSCHRIFT**

(45) Veröffentlichungstag und Bekanntmachung des Hinweises auf die Patenterteilung:
**17.08.2005 Patentblatt 2005/33**

(21) Anmeldenummer: 02730229.8

(22) Anmeldetag: **01.05.2002**

(51) Int Cl.⁷: **B01J 20/12**, B01J 20/30, B01J 21/16

(86) Internationale Anmeldenummer:
**PCT/EP2002/004779**

(87) Internationale Veröffentlichungsnummer:
**WO 2002/100533 (19.12.2002 Gazette 2002/51)**

(54) **VERFAHREN ZUR HERSTELLUNG VON ADSORPTIONSMITTELGRANULATEN AUF DER BASIS VON SÄUREAKTIVIERTEN SCHICHTSILIKATEN UND DEREN VERWENDUNG ALS KATALYSATOR**

METHOD FOR THE PRODUCTION OF ADSORPTION GRANULES BASED ON ACID-ACTIVATED LAMINAR SILICATES AND USE THEREOF AS CATALYST

PROCEDE DE PRODUCTION DE GRANULES D'ADSORBANTS A BASE DE SILICATES LAMELLAIRES ACTIVES PAR LIXIVIATION ACIDE ET LEUR UTILISATION COMME CATALYSEUR

(84) Benannte Vertragsstaaten:
**AT BE CH CY DE DK ES FI FR GB GR IE IT LI LU MC NL PT SE TR**

(30) Priorität: **08.06.2001 DE 10127927**

(43) Veröffentlichungstag der Anmeldung:
**17.03.2004 Patentblatt 2004/12**

(73) Patentinhaber: **SÜD-CHEMIE AG**
**80333 München (DE)**

(72) Erfinder: **FLESSNER, Uwe**
**82061 Neuried (DE)**

(74) Vertreter: **Westendorp, Michael, Dr. et al**
**Patentanwälte**
**Splanemann Reitzner**
**Baronetzky Westendorp**
**Rumfordstrasse 7**
**80469 München (DE)**

(56) Entgegenhaltungen:
**WO-A-01/34295          DE-A- 4 405 876**
**DE-A- 4 405 878        DE-A- 19 601 861**
**US-A- 2 485 626        US-A- 5 208 195**

## Beschreibung

Die Erfindung betrifft ein Verfahren zur Herstellung von Adsorptionsmittelgranulaten auf der Basis von säureaktivierten Schichtsilicaten, nach diesem Verfahren erhältliche Adsorptionsmittelgranulate sowie deren Verwendung als Katalysator zur Entfernung von Olefinen aus Aromaten oder Aromatengemischen.

Industriell bedeutsame Aromaten, wie z.B. Benzol, Toluol oder Xylol werden typischerweise durch sog. Reforming aus Naphthaschnitten hergestellt. Das Reforming erzeugt dabei aus dem vormals ungesättigten Naphtha ein Gemisch aus verschiedenen aromatischen und ungesättigten Verbindungen. Durch geeignete Extraktions- bzw. Destillationsverfahren werden die aromatischen Fraktionen von den aliphatischen getrennt. Die hierbei verwendeten Trennungsverfahren erlauben es jedoch nicht, die olefinischen Bestandteile vollständig aus dem Aromatenstrom zu entfernen. Es verbleiben geringe Mengen Olefine in den Aromaten, die, obwohl ihr Anteil unter 1% liegt, in nachfolgenden Veredelungsprozessen stören. Da die unerwünschten Olefine die gleichen Siedepunkte aufweisen wie die Aromaten; ist eine destillative Abtrennung nicht möglich.

Als wirtschaftlichstes Verfahren zur Entfernung dieser Olefine hat sich weltweit die katalytische Behandlung mit aktivierten Erdalkali-Alumosilicaten, wie z.B. säureaktivierten Smectiten durchgesetzt. Die Smectite werden hierbei in einer Feingranulatform (Partikelgröße < 1,0 mm) in einem Festbett eingesetzt. Der Aromatenstrom wird bei ca. 150 - 220°C unter Drücken um 10 bar über dieses Festbett geleitet, wobei die Katalysatorgranulate die unerwünschten Olefine in höhersiedende Produkte umwandeln. Nachgeschaltete Destillationskolonnen sind dann in der Lage, Aromaten von den verbleibenden Olefinprodukten zu separieren.

Als geeignete Katalysatoren werden in der Regel säureaktivierte Mineralien verwendet. Die hierbei Einsatz findenden Mineralien werden zumeist aus der Gruppe der Smectite oder der Palygorskite (Attapulgite) ausgewählt. Die Säureaktivierung kann durch einfaches Besprühen mit einer nachgeschalteten thermischen Behandlung erfolgen oder ein aufwendiger Säureextraktionsprozess sein, bei dem das Mineral in Suspension mit einer zumeist mineralischen Säure bei hohen Temperaturen behandelt wird. Hierbei findet eine Extraktion von löslichen Komponenten statt, wodurch die Oberfläche und das Porenvolumen des Minerals erhöht werden. Aufgrund ihrer höheren Reinigungsleistung (katalytischen Aktivität) haben sich insbesondere die säureausgelaugten Produkte auf Basis von montmorillonithaltigen Tonen durchgesetzt. Dies trifft umso mehr zu, als in den vergangenen Jahren besonders schwer zu behandelnde Aromatenströme verarbeitet werden mussten. Diese schwer zu behandelnden Aromatenströme sind hauptsächlich auf das Behandeln von schweren Rohölen ("bottom of the barrel") bzw. durch den Einsatz neuer Reformerkatalysatoren, deren hohe Aromatisierungsleistung zu hohen Anteilen an Polyaromaten im Aromatenstrom führt, zurückzuführen. Diese Aromatenströme verursachen nun ein schnelles Deaktivieren der säureaktivierten Produkte, was sich in Standzeitverkürzungen bis zu 50 - 70% bemerkbar macht.

Die höheren Anforderungen der Aromatenströme an die zu verwendenden Olefinentfernungskatalysatoren erfordert die Entwicklung besonders aktiver Katalysatoren. Eine verbesserte Aktivität konnte durch Verwendung spezieller Montmorillonit-Tonsorten sowie durch Einführung von speziellen Aktivierungsverfahren erzielt werden.

Diese neuen hochaktiven Katalysatoren lassen sich aber nach üblichen Granulationsverfahren nur zu strukturell schwachen Granulaten verformen. Die schwache Struktur der Granulate führt dazu, dass während der Befüllung eines typischen Reaktors eine Vielzahl der Granulate zu feinkörnigem bis staubigem Material zerbricht, welches beim späteren Betrieb negative Effekte durch Austrag dieser Feinstoffe verursacht.

Die DE 44 05 878 Al beschreibt ein Verfahren zur Herstellung von Adsorptionsmittelgranulaten, wobei säureaktivierte Silicate getrocknet und das resultierende Produkt zerkleinert wird. Das zerkleinerte Produkt wird anschließend nach dem Befeuchten mit Wasser oder einer wasserhaltigen Flüssigkeit unter Bildung von Klumpen verknetet. Hierbei werden z.B. Eirich-Intensivmischer oder Extruder verwendet. Die bei diesen Verfahren hergestellten Formkörper werden anschließend über Brecher auf 0,25 - 1 mm gebrochen.

Ein wichtiger Schritt des dort beschriebenen Verfahrens ist die Zerkleinerung des getrockneten aktivierten Silicates, wobei bevorzugt eine Teilchengröße von 5 - 80 μm eingestellt wird. Nachteilig an diesem Verfahren ist, dass die damit verbundene zweifache Trocknung des Ausgangsmaterials das Verfahren vom energetischen Standpunkt aus unwirtschaftlich macht. Es wird keine deutliche Verdichtung der Granulate erzielt. Beim Brechen der im Primärschritt gebildeten Granulate wird naturgemäß Staub gebildet, der in den Prozess rückgeführt werden muss.

Die EP-B-0 702 596 beschreibt katalytische Materialien, die Makrokugeln aus Schichtsilicat enthalten, sowie ein Verfahren zu deren Herstellung. Durch die strenge Kontrolle des Prozesses wird eine Ausbeute von 90% erreicht. Die kugelförmigen Produkte sind stabil und aufgrund der Abwesenheit von Bruchkanten neigen sie nicht zum Staubbilden während der Befüllung. Die in der EP-B-0 702 596 durchgeführte Aufbaugranulation ist jedoch nicht in der Lage, eine deutliche Verdichtung bzw. Schüttgewichtserhöhung zu erzielen.

Nach einem alternativen Herstellungsverfahren werden Bindersysteme verwendet, wie sie in der DE 44 05 876 A1 beschrieben sind. Die Aktivkomponente wird hierbei unter Zuhilfenahme von Bindersystem geformt. Der Anteil des Binders liegt typischerweise im Bereich von 15 - 25%, kann jedoch auch 50% erreichen. Mit diesem Verfahren können sehr stabile Produkte mit hohem Schüttgewicht hergestellt werden. Durch den Einsatz des Binders wird aber Inertmaterial eingeführt, so dass die installierte "effektive" Katalysatormasse reduziert wird.

Es bestand nun die Aufgabe, hochaktive Adsorptionsmittelgranulate bereitzustellen, die die Nachteile des Standes der Technik vermeiden, und insbesondere eine hohe katalytische Aktivität bei hoher Druck- bzw. Abriebfestigkeit und chemischer bzw. thermischer Beständigkeit aufweisen.

Diese Aufgabe wird durch ein Verfahren gemäß Anspruch 1 gelöst. Vorteilhafte Ausführungsformen ergeben sich aus den Unteransprüchen.

Im Rahmen der vorliegenden Erfindung kann allgemein jedes mit Säure aktivierbare Dreischichtsilicat verwendet werden. Dreischichtsilicat kann ein natürliches oder synthetisches Dreischichtsilicat sein. Das Dreischichtsilicat ist vorzugsweise aus der Gruppe der Smectite (wie Montmorillonit, Hectorit, Antigorit, Nontronit, Beidellit oder Saponit), Vermiculite, Illite und Glimmer ausgewählt.

Andere brauchbare Dreischichtsilicate sind Sepiolith, Attapulgit (Palygorskit), Stevensit oder Chlorit, unter den montmorillonithaltigen Mineralien sind insbesondere Bentonit und Fuller's Earth zu nennen, die je nach Fundort unterschiedlich zusammengesetzt sein können.

Das Dreischichtsilicat kann chemisch und/oder thermisch modifiziert sein. Unter einer chemischen Modifizierung versteht man erfindungsgemäß nicht nur die Säureaktivierung, sondern auch eine zusätzliche chemische Behandlung, z.B. eine Silanisierung der Oberfläche.

Bei der Säureaktivierung wird das Dreischichtsilicat mit einer beliebigen anorganischen und/oder organischen Säure, vorzugsweise einer Mineralsäure, wie Salzsäure, Phosphorsäure oder Schwefelsäure, umgesetzt. Die Umsetzung kann bei erhöhten Temperaturen erfolgen. Vorzugsweise wird die saure Aktivierungsflüssigkeit, die jetzt die Salze von ein- oder mehrwertigen Kationen aus den Silicaten enthält, von dem säureaktivierten Silicat (im allgemeinen durch Filtrieren) abgetrennt. Der erhaltene feuchte Filterkuchen wird dann ggf. säurefrei gewaschen.

Dabei wird die Säureaktivierung erfindungsgemäß so durchgeführt, dass mindestens 10%, vorzugsweise mindestens etwa 15%, und insbesondere mindestens etwa 20% des im Ausgangsmaterial vorhandenen Aluminiumoxids ausgelaugt bzw. entfernt werden. Diese Art der Säureaktivierung wird auch als Säure(aus)laugung bezeichnet, im Gegensatz zur oberflächlichen Säureaktivierung mit geringeren Säuremengen.

Die thermische Behandlung schließt eine Trocknung, gegebenenfalls eine Vakuumtrocknung oder Sprühtrocknung und/oder eine Calcinierung ein. Die thermische Behandlung kann unter oxidierenden, reduzierenden oder inerten Bedingungen sowie in Anwesenheit von Wasserdampf durchgeführt werden. Die thermische und die chemische Modifizierung können in geeigneter Weise kombiniert sein. Vor und/oder nach der chemischen Behandlung kann gegebenenfalls eine thermische Behandlung durchgeführt werden. Die chemische Behandlung kann bei erhöhter Temperatur und/oder bei erhöhtem Druck durchgeführt werden.

Die erfindungsgemäßen Adsorptionsmittelgranulat-Formkörper können auch Aktivator- und/oder Promotorsubstanzen enthalten. Zur Aktivierung bzw. Promotierung verwendet man mindestens eine Verbindung der Alkali- und/ oder Erdalkalimetalle und/oder Verbindungen der Elemente der Gruppen (nach IUPAC alt) IB (wie Cu, Ag oder Au), IIB (wie Zn und Cd), IIIA (wie Sc oder Y) oder IIIB (wie E, Al, Ga, In oder Tl), IVA (wie Ti, Zr oder Hf), IVB (wie Si, Ge, Sn), oder der Lanthanoiden oder Actinoiden, VA (wie V, Nb oder Ta), VB (wie P, As, Sb oder Bi), VIA (wie Cr, Mo, oder W), VIB (wie S, Se), VIIA (wie Fe, Co, Ni, Ru, Rh, Pd, Os, Ir oder Pt).

Wesentlich ist, dass bei dem erfindungsgemäßen Verfahren durch hochkompaktierende Formgebungsschritte eine erhebliche Erhöhung der Schüttdichte des Adsorptionsmittels bzw. Katalysatormaterials erfolgt. Dabei kann jede geeignete Vorrichtung verwendet werden, die eine Erhöhung der Schüttdichte um mindestens 10% bewirken kann. Dies ist z.B. bei üblichen Extrudern oder Brikettierpressen nicht der Fall. Geeignete Vorrichtungen sind dem Fachmann geläufig oder können anhand routinemäßiger Versuche bestimmt werden.

Die Schüttdichte wurde folgendermaßen bestimmt: Ein 500ml-Messzylinder, der an der 500 ml Marke abgeschnitten wurde, wird zunächst leer gewogen. Darauf wird ein Pulvertrichter mit einer Öffnung von etwa 15cm und einer Auslassöffnung von etwa 3cm aufgesetzt und innerhalb von etwa 5 Sekunden mit dem Granulat gefüllt. Danach wird der Pulvertrichter vom Messzylinder abgenommen, und zwar so, dass das darin befindliche Granulat einen überstehenden Kegel bildet. Dieser wird mit einem breiten Spatel entlang der Schnittkante des Messzylinders abgestreift. Der gefüllte Messzylinder wird außen von anhängenden Körnern oder Staub befreit und erneut gewogen. Das Schüttgewicht wird wie folgt berechnet:

$$\text{Schüttgewicht (g/l)} = 2 \times \text{Gewicht netto (g/500ml)}.$$

Nach einer bevorzugten Ausführungsform erfolgt die hochkompaktierende Behandlung mit einer Tablettierpresse oder einer Ringscheibenpresse. Die Formgebung wird dabei derart durchgeführt, dass direkt Formstücke der Größe 1 - 5 mm, insbesondere 1 - 4 mm, gebildet werden. Nach einer vorteilhaften erfindungsgemäßen Ausführungsform wird die Schüttdichte (im Unterschied zu den Formgebungsverfahren des Standes der Technik) um bis zu 25 - 30% erhöht.

Überraschenderweise bewirkt weder die starke Kompaktierung noch die Vergrößerung des Formkörpers Aktivitätsverluste des Katalysators. Durch die Erhöhung der Schüttdichte kann die Standzeit des Katalysators bei gegebenem Reaktorvolumenum die gleiche Größenordnung verlängert werden.

Nach einer vorteilhaften Ausführungsform wird zur Einstellung des für die Formgebung benötigten Wassergehaltes der Filterkuchen, der aus der sauren Aktivierung resultiert und typischerweise einen Feststoffgehalt von etwa 40% aufweist, durch Zumischen von getrocknetem Filterkuchen und/oder von nicht säureaktiviertem vorgetrocknetem Schichtsilicat (Rohton) eingestellt. Dadurch ist eine ökonomische und kostengünstige Verfahrensführung möglich.

Nach einer weiteren bevorzugten erfindungsgemäßen Ausführungsform werden die Formkörper nach der Formgebung getrocknet, das Oberkorn gebrochen und zum Siebschritt rückgeführt. Die abgesiebten Feinanteile werden vorteilhaft in das Verfahren zurückgeleitet und vorzugsweise zusammen mit dem getrockneten säureaktivierten Schichtsilicat bzw. Rohton nochmals zerkleinert.

Ein weiterer Aspekt der vorliegenden Erfindung betrifft die Verwendung der erfindungsgemäßen Granulate bzw. Formkörper als Adsorptionsmittel oder Katalysator für katalytische Reaktionen.

Besonders eignen sie sich als Katalysator für die Behandlung von Aromaten oder Aromatengemischen, vorzugsweise zur Entfernung von Olefinen aus Aromaten oder Aromatengemischen.

Solche olefinischen Verunreinigungen können, wie eingangs erläutert, häufig aus Gemischen aromatischer Kohlenwasserstoffe wegen der Ähnlichkeit der Siedepunkte nicht ohne weiteres destillativ abgetrennt werden. Mit den erfindungsgemäßen Adsorptionsmittelgranulaten werden die Olefine an den sauren Zentren der säureaktivierten Silicate bei erhöhten Temperaturen oligomerisiert, wodurch sich ihre Siedepunkte erhöhen, so dass eine destillative Abtrennung von den Aromaten möglich ist. Dabei wird das Aromatengemisch gewöhnlich bei Temperaturen zwischen etwa 150 und 220°C über ein Schüttbett des erfindungsgemäßen Adsorptionsmittels geleitet.

Ferner können die erfindungsgemäßen Adsorptionsmittelgranulate auch als Katalysatoren für andere säurekatalysierte Reaktionen eingesetzt werden. Zu diesen Reaktionen gehören Veretherungen durch Umsetzung von Alkoholen oder Epoxiden, Veresterungen durch Umsetzung von Säuren bzw. Säureanhydriden mit Alkoholen, Epoxiden und Olefinen, Isomerisierungen , Herstellung von alkylaromatischen Verbindungen durch Umsetzung von aromatischen Verbindungen mit Alkoholen bzw. Olefinen, Hydratisierungen von Olefinen und Dehydratisierungen von Alkoholen, Alkoxylierung und Acetalisierung von ungesättigten Aldehyden, Herstellung von alkylierten aromatischen Aminen und ähnliche säurekatalysierte Reaktionen.

Wie vorstehend ausgeführt, weisen die nach dem erfindungsgemäßen Verfahren hergestellten Adsorptionsmittelgranulate bzw. -formkörper eine hervorragende katalytische Aktivität bei höher Schüttdichte und hervorragender Standzeit auf.

Ein weiterer Aspekt der vorliegenden Erfindung betrifft daher ein Adsorptionsmittel, insbesondere einen Katalysator, erhältlich nach einem Verfahren gemäß einem der Ansprüche 1 bis 7. Das erfindungsgemäße Adsorptionsmittel weist ein Schüttgewicht von 700 bis 900 g/l, insbesondere 700 bis 800 g/l, und eine Formkörpergrösse von 1 bis 5 mm, insbesondere 1 bis 4 mm auf.

Die Erfindung ist durch die nachstehenden Beispiele erläutert:

## Beispiele

Vergleichsmaterialien

Die erfindungsgemäßen Produkte werden im Vergleich zu den am Markt erhältlichen Produkten Tonsil CO 616 G und Tonsil CO 616 GS (Süd-Chemie AG) verglichen.

Tonsil CO 616 G ist ein weltweit eingesetzter Katalysator zur Behandlung von Benzol und Toluol-Fraktionen.

Tonsil CO 616 GS ist ein typischer Vertreter der neuen hochaktiven Katalysatorgeneration. Er wird bevorzugt zur Reinigung von schwer zu behandelnden Aromatenströmen eingesetzt.

## Beispiel 1

Handelsübliches Tonsil CO 616 GS wird in einer Mühle zu Staub < 100 μm vermahlen und anschließend mit Wasser auf einen Wassergehalt von 35% eingestellt. Die homogene Mischung wird dann in einer sogenannten Ringscheibenpresse mit einem Lochdurchmesser von 3 mm unter stark kompaktierenden Bedingungen zu Strangpresslingen verformt. Ein mitlaufendes Schneidewerkzeug kürzt während der Formgebung die Strangpresslinge bei ca. 4 mm ab. Das feuchte Formstück wird in einem Trommeltrockner getrocknet und auf 1 - 4 mm gesiebt. Das hierbei resultierende Oberkorn wird gebrochen und in die Sieberei rückgeführt. Der dabei anfallende Staub ist vernachlässigbar, wird aber in die Produktionslinie rückgeführt.

**Beispiel 2**

Das rückgefeuchtete pulverisierte Material aus Beispiel 1 wird zusätzlich mit 10 Gew.-% pulverisiertem, nicht aktivierten Montmorillonit versetzt. Die Formgebung, die Trocknung und das Sieben verlaufen wie in Beispiel 1 beschrieben.

**Beispiel 3**

Das Beispiel 2 wird wiederholt, wobei 20% Montmorillonit-Ton (vgl. Beisp. oben) zugegeben wird.

Tabelle 1

| Die Tabelle 1 gibt Analysenwerte der resultierenden Produkte an: | | | | | |
|---|---|---|---|---|---|
| | Tonsil CO 616 G | Tonsil CO 616 GS | BSP 1 | BSP 2 | BSP 3 |
| BET ($m^2/g$) | 205 | 290 | 287 | 280 | 275 |
| Porenvolumen (ml/g) | 0,220 | 0,450 | 0,420 | 0,390 | 0,375 |
| Schüttgewicht (g/l) | 680 | 570 | 750 | 780 | 800 |
| Partikelfraktion (mm) | 0,25 - 1,0 | 0,25 - 1,0 | 1 - 4 | 1 - 4 | 1 - 4 |

Die Analysenwerte der spezifischen Oberfläche sowie des Porenvolumens der Beispielprodukte belegen eindrucksvoll, dass die starke Kompaktierung nicht zur Zerstörung der Porosität des Systems führt. Der Vergleich der Schüttgewichtswerte macht deutlich, dass trotz gleicher Tonbasis wie Tonsil CO 616 GS deutlich höhere Schüttgewichte in den Beispielprodukten erzielt werden können. Diese liegen sogar höher als die des gering porösen Materials Tonsil CO 616 G.

**Beispiel 4 (Anwendungsbeispiel)**

In einem Laborversuch soll die Reinigungsleistung der Produkte bei der Behandlung eines technischen Xylols untersucht werden. In einem Laborteststand wird eine Edelstahl-HPLC-Säule (10 mm Innendurchmesser, 150 ml Länge) mit 10 ml Granulat der Beispiele 1 - 3 sowie der Vergleichsmaterialien gefüllt. Um gleiche Strömungsbedingungen zu verwirklichen, wurden alle Granulate auf eine Fraktion von 0,25 - 0,5 mm gebrochen. Über die gefüllte HPLC-Säule wird ein technisches Xylol geleitet, wobei die Temperatur mit Hilfe eines thermostatisierten Ölbades auf 175°C geregelt wird. Um eine flüssige Phase zu gewährleisten, wird mittels Druckregler ein Druck von 10 bar eingestellt. Die Förderleistung der HPLC-Pumpe wird auf eine Raumgeschwindigkeit von 6h$^{-1}$ gestellt. In regelmäßigen Abständen werden Proben nach der Reinigung genommen und der Bromindex als Maß des Olefinanteils im Produkt bestimmt. Das Ausgangsxylol hatte einen Bromindex von 512 mg/100 g; durch die Behandlung mit den Granulaten sank dieser für mehrere Tage unter 5 mg/100 g. Der Versuch wurde so lange durchgeführt, bis der Bromindex des behandelten Xylols 40 mg/100 g erreichte. An dieser Stelle wurde die Laufzeit notiert und der Versuch mit einem frischen Katalysator wiederholt.

Tabelle 2

| Die Tabelle 2 gibt die aus verschiedenen Versuchswiederholungen gemittelten Ergebnisse wieder: | | | | | |
|---|---|---|---|---|---|
| | Tonsil CO 616 G | Tonsil CO 616 GS | BSP 1 | BSP 2 | BSP 3 |
| Katalysatorvolumen (ml) | 10 | 10 | 10 | 10 | 10 |
| Einwaage (g) | 6,65 | 5,73 | 7,50 | 7,79 | 7,93 |
| Laufzeit (h) | 288 | 343 | 445 | 470 | 465 |

Die Ergebnisse des Versuches 4 belegen eindrucksvoll, dass die erfindungsgemäßen Produkte die Standzeit der Katalysatoren deutlich verlängern können.

Beispiel 5 (Technische Anwendung)

In einem Seitenstromreaktor einer typischen Aromatenanlage in der petrochemischen Industrie wurde das Produkt aus Beispiel 1 (Partikelfraktion 1 - 4 mm) gegen das Produkt Tonsil CO 616 GS (Partikelfraktion 0,25 - 1 mm) abgetestet. Der Seitenstromreaktor hatte ein Volumen von 50 1; durch diesen Reaktor wurde ein technisches Xylol mit einem Bromindex von 820 mg/100 g unter einem Druck von ca. 10 bar geleitet. Die Temperatur bei dieser Versuchsführung betrug 180°C und die Raumgeschwindigkeit wurde auf 1,0 h$^{-1}$ geregelt. Der direkte Vergleich der Standzeiten der getesteten Katalysatorsysteme wies einen Vorteil für das erfindungsgemäße Produkt um 25% entsprechend der höheren Einwaage aus. Somit konnte in dieser Versuchsserie gezeigt werden, dass auch die großen hochkompaktierten Formkörper eine verbesserte Reinigungsleistung aufweisen können.

**Patentansprüche**

1. Verfahren zur Herstellung von Adsorptionsmittelgranulaten auf der Basis von säureaktivierten Dreischichtsilicaten, wobei durch die Säureaktivierung mindestens 10% des im Ausgangmaterial vorhandenen Aluminiumoxids entfernt werden, und wobei das aus der Säureaktivierung erhältliche Filtergut ohne Zusatz eines Bindemittels über hochkompaktierende Formgebungsschritte zu Formstücken der Größe 1 bis 5 mm verformt und eine Erhöhung der Schüttdichte um mindestens 10% bewirkt wird, sodass nach der Kompaktierung das Adsorptionsgranulat eine Schüttdichte im Bereich von 700 - 900 g/l aufweist, und nach der Kompaktierung keine Calcinierung des Adsorptionsmittelgranulats durchgeführt wird.

2. Verfahren nach Anspruch 1, **dadurch gekennzeichnet, dass** durch die Säureaktivierung bzw. Säureauslaugung mindestens etwa 15%, insbesondere mindestens etwa 20% des im Ausgangsmaterial vorhandenen Aluminiumoxids ausgelaugt bzw. entfernt werden.

3. Verfahren nach einem der Ansprüche 1 oder 2, **dadurch gekennzeichnet, dass** durch die hochkompaktierenden Formgebungsschritte eine Erhöhung des Schüttgewichts von mindestens etwa 15%, vorzugsweise mindestens etwa 20%, insbesondere mindestens etwa 25%, bewirkt wird.

4. Verfahren nach einem der vorstehenden Ansprüche, **dadurch gekennzeichnet, dass** man das aus der Säureaktivierung erhältliche Filtergut mit zumindest teilweise getrocknetem Filtergut oder dem zur Säureaktivierung eingesetzten Schichtsilicat mit geringerem Wassergehalt abmischt, bevor über die hochkompaktierenden Formgebungsschritte verformt wird.

5. Verfahren nach einem der vorstehenden Ansprüche, **dadurch gekennzeichnet, dass** die hochkompaktierenden Formgebungsschritte eine Behandlung mit einer Tablettenpresse oder einer Ringscheibenpresse umfassen.

6. Verfahren nach einem der vorstehenden Ansprüche, **dadurch gekennzeichnet, dass** die Adsorptionsmittelgranulate zu Formstücke der Größe 0,5 - 5 mm, insbesondere 1 - 4 mm, geformt werden, vorzugsweise direkt im hochkompaktierenden Formgebungsschritt.

7. Verfahren nach einem der vorstehenden Ansprüche, **dadurch gekennzeichnet, dass** als Schichtsilicate montmorillonithaltige Schichtsilicate, insbesondere Bentonite eingesetzt werden.

8. Verwendung eines Adsorptionsmittelgranulats, erhältlich nach einem Verfahren gemäß Anspruch 1 bis 7 als Katalysator für katalytische Reaktionen.

9. Verwendung nach dem vorstehenden Anspruch als Katalysator für die Behandlung von Aromaten oder Aromatengemischen, insbesondere zur Entfernung von Olefinen aus Aromaten oder Aromatengemischen.

10. Adsorptionsmittel, insbesondere Katalysator, erhältlich nach einem Verfahren gemäß einem der Ansprüche 1 bis 6.

**Claims**

1. A method for the production of adsorbent granules based on acid-activated three-layered silicates, wherein at least 10 % of the aluminium oxide present in the starting material are removed by the acid activation, and wherein the filter material which can be obtained from the acid activation is shaped without the addition of a binder by way of high-compaction forming steps to form mouldings of a size of 1 to 5 mm and an increase in the bulk density of at least 10 % is brought about so that, after the compacting, the adsorption granular material has a bulk density in the range of from 700 to 900 g/l and after the compacting no calcination of the adsorbent granular material is carried out.

2. A method according to Claim 1, **characterised in that** as a result of the acid activation or the acid leaching at least approximately 15 %, in particular at least approximately 20 %, of the aluminium oxide present in the starting material are leached out or removed.

3. A method according to either Claim 1 or Claim 2, **characterised in that** as a result of the high-compaction forming steps an increase in the bulk density of at least approximately 15 %, preferably at least approximately 20 %, in particular at least approximately 25 %, is brought about.

4. A method according to any one of the preceding Claims, **characterised in that** the filter material which can be obtained from the acid activation is mixed with at least partly dried filter material or with the layered silicate having lower water content used for the acid activation before it is shaped by way of the high-compaction forming steps.

5. A method according to any one of the preceding Claims, **characterised in that** the high-compaction forming steps comprise a treatment with a tabletting press or a ring-disc press.

6. A method according to any one of the preceding Claims, **characterised in that** the adsorbent granules are shaped to form mouldings of a size of 0.5 to 5 mm, in particular 1 to 4 mm, preferably directly in the high-compaction forming step.

7. A method according to any one of the preceding Claims, **characterised in that** layered silicates containing montmorillonite are used as layered silicates, in particular bentonites.

8. Use of an adsorbent granular material which can be obtained in accordance with a method according to Claims 1 to 7 as a catalyst for catalytic reactions.

9. Use according to the preceding Claim as a catalyst for the treatment of aromatics or aromatic mixtures, in particular for the removal of olefins from aromatics or aromatic mixtures.

10. An adsorbent, in particular a catalyst, which can be obtained in accordance with a method according to any one of Claims 1 to 6.


**Revendications**

1. Procédé de préparation de granulés d'un adsorbant à base de silicates à trois couches activés à l'acide, dans lequel au moins 10% de l'oxyde d'aluminium présent dans la matière de départ sont éliminés par l'activation à l'acide, et dans lequel le filtrat obtenu lors de l'activation à l'acide est façonné en objets moulés d'une taille de 1 à 5 mm dans des étapes de façonnage à haute compression sans addition de liant, et une augmentation de la masse volumique apparente d'au moins 10% est obtenue, de sorte qu'après la compression, les granulés d'adsorbant présentent une masse volumique apparente dans la gamme de 700 à 900 g/L et après la compression, aucune calcination des granulés d'adsorbant n'est réalisée.

2. Procédé selon la revendication 1, **caractérisé en ce qu'**au moins 15% environ, en particulier au moins 20% environ de l'oxyde d'aluminium présent dans la matière de départ sont lixiviés ou éliminés par l'activation à l'acide.

3. Procédé selon l'une des revendications 1 ou 2, **caractérisé en ce que** les étapes de façonnage à haute compression entraînent une augmentation de la masse volumique apparente d'au moins 15% environ, de préférence d'au moins 20% environ, en particulier d'au moins 25% environ.

4. Procédé selon l'une quelconque des revendications précédentes, **caractérisé en ce que** l'on mélange le filtrat obtenu lors de l'activation à l'acide avec un filtrat au moins partiellement séché ou avec le silicate à couches ayant une faible teneur en eau mis en oeuvre dans l'activation à l'acide, avant de réaliser le façonnage dans les étapes de façonnage à haute compression.

5. Procédé selon l'une quelconque des revendications précédentes, **caractérisé en ce que** les étapes de façonnage à haute compression comprennent un traitement avec une pastilleuse ou une presse à disques.

**6.** Procédé selon l'une quelconque des revendications précédentes, **caractérisé en ce que** les granulés d'adsorbant sont façonnés en objets moulés d'une taille de 0,5 à 5 mm, en particulier de 1 à 4 mm, de préférence directement dans l'étape de façonnage à haute compression.

**7.** Procédé selon l'une quelconque des revendications précédentes, **caractérisé en ce que** l'on met en oeuvre, en tant que silicates à couches, des silicates à couches contenant de la montmorillonite, en particulier des bentonites.

**8.** Utilisation d'un granulé d'adsorbant pouvant être obtenu au moyen d'un procédé selon les revendications 1 à 7, en tant que catalyseur pour des réactions catalytiques.

**9.** Utilisation selon la revendication précédente en tant que catalyseur pour le traitement de composés aromatiques ou de mélanges de composés aromatiques, en particulier pour l'élimination d'oléfines de composés aromatiques ou de mélanges de composés aromatiques.

**10.** Adsorbant, en particulier catalyseur, pouvant être obtenu au moyen d'un procédé selon l'une des revendications 1 à 6.